(19) Europäisches Patentamt
European Patent Office
Office européen des brevets

(11) **EP 3 839 885 A1**

(12) **EUROPEAN PATENT APPLICATION**

(43) Date of publication:
**23.06.2021 Bulletin 2021/25**

(51) Int Cl.:
**G06T 7/00** (2017.01)

(21) Application number: **20215060.3**

(22) Date of filing: **17.12.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **19.12.2019 CN 201911313396**

(71) Applicant: **Shanghai Aitrox Technology Co., Ltd Shanghai 202150 (CN)**

(72) Inventors:
• YE, Dexian
  Xuhui District, shanghai 200233 (CN)
• FANG, Qu
  Xuhui District, shanghai 200233 (CN)
• JIANG, Chenxi
  Xuhui District, Shanghai 200233 (CN)

(74) Representative: **EDP Patent Attorneys B.V.**
**Bronland 12-E**
**6708 WH Wageningen (NL)**

(54) **REAL-TIME PATHOLOGICAL MICROSCOPIC IMAGE COLLECTION AND ANALYSIS SYSTEM, METHOD AND DEVICE AND MEDIUM**

(57) A system and method capable of implementing real-time scanning and rapidly and efficiently analyzing a pathological state are provided. The system includes: a microscopic image collection device; an image analysis device, connected with the microscopic image collection device and configured to acquire at least one microscopic image block in real time, input the at least one microscopic image block to a trained neural network model for analyzing to obtain at least one microscopic image block analysis result; and a display device, connected with the image analysis device and outputting and displaying the at least one microscopic image block analysis result. The present invention also provides a method or medium corresponding to the device.

**Fig. 3**

## Description

### Cross-reference to Related Applications

The present invention claims priority of Chinese Patent Application No. 201911313396.4, filed to China Patent Office on December 19, 2019. Contents of the present disclosure are hereby incorporated by reference in entirety of the Chinese Patent Application.

### Technical Field

The present invention relates to a real-time image collection and analysis technology, which belongs to the technical field of micropathology aided diagnosis, and in particular to a real-time pathological microscopic image collection and analysis system, a real-time pathological microscopic image collection and analysis method and a real-time pathological microscopic image collection and analysis device and a non-transitory computer-readable storage medium.

### Background

At present, an aided diagnosis method for pulmonary diseases is usually Computer Tomography (CT)-guided needle biopsy, which is a relatively common method for diagnosis of pulmonary diseases. A sample Rapid On-Site Evaluation (ROSE) technology, commonly used in this field at present, refers to that a cell pathologist rapidly examines sample cells on site and evaluates the quality of a fine needle aspiration smear and a bioptic imprint. An examiner may learn about whether there are enough samples or not by ROSE to determine whether more samples are required to be collected or not, thereby avoiding a patient from repeated puncturing, ensuring that enough samples are collected at one time and simultaneously implementing preliminary diagnosis by ROSE to provide a required analysis result for subsequent disease diagnosis processing.

Specifically, a transbronchial bioptic imprint and a fine needle aspiration smear are samples obtained by forceps biopsy and brush biopsy of a bronchial or pulmonary lesion or transbronchial needle biopsy of a hilar or mediastinal lymph node in transbronchial lung biopsy, and a physician performs ROSE on the samples. The physician may fix and stain slides and then microscopically observe pulmonary cells. However, the technology provided in related art has the following technical defects: since a physician that performs ROSE on a transbronchial bioptic imprint and a fine needle aspiration smear is required to be a pathologist and it is difficult for a respiratory physician to complete ROSE, the transbronchial bioptic imprint and the fine needle aspiration smear are required to be sent to the pathology department, this process prolongs time for ROSE, but it is usually a respiratory physician performing an operation on a patient on an operating table and there is an urgent need for obtaining a testing and analysis result in real time; and time is precious for the patient on the operating table, but an existing microscope scanner may generate a complete large microscopic image after completing scanning the whole transbronchial bioptic imprint and fine needle aspiration smear and scanning and image processing and analysis cannot be implemented at the same time in a scanning process, prolonging analysis and processing time and bringing difficulties to rapid disease diagnosis. In a word, there are two problems at present: one is high time consumption caused by a requirement of sending to the pathology department for manual examination, and the other is time waste caused by the fact that examination may be started after the large image is collected.

In practice, pathological microscopic diagnosis is applied to many disease detection and treatment scenarios, for example, micropathologic diagnosis of pulmonary cell pathology, cervical cell pathology, breast pathology or thyroid pathology, and there is also made the requirement of sending to the pathology department for manual examination. An existing method is relatively high in time consumption and cannot meet a diagnosis requirement in emergency.

### Summary

At least some embodiments of the present invention provides a system or method capable of implementing real-time scanning and rapidly and efficiently analyzing a pathological state, thereby effectively overcoming the abovementioned technical defects and solving the technical problems.

In an embodiment of the present invention, a real-time pathological microscopic image collection and analysis system is provided, which is configured to perform microscopic image collection and real-time analysis and diagnosis on a sample, the sample including at least one human cell or tissue. The system includes a microscopic image collection device, an image analysis device and a display device.

The microscopic image collection device includes: an object stage, configured to carry the sample; a camera, configured to shoot the sample to obtain a microscopic image; and a control element, configured to control relative movement of the object stage and the camera and control the camera to sequentially shoot multiple positions of the sample to obtain microscopic image blocks, each microscopic image block referring to a region, which may be shot by the camera each time, of the sample. Since a size of the sample is usually larger than a width of a microscopic field shot by the camera, a microscope is required to sequentially shoot the sample in a blocking manner, one microscopic image block being obtained by shooting once.

The image analysis device is connected with the microscopic image collection device and configured to acquire at least one microscopic image block in real time, input the obtained at least one microscopic image block to a trained neural network model in real time for analyzing to obtain at least one microscopic image block analysis result. Each of the at least one microscopic image block analysis result include an analysis result, corresponding to each microscopic image block, of whether sample cells have a pathological abnormality or not. That is, when the sample has the pathological abnormality, the at least one microscopic image block in the sample may include information about whether the sample cells have the pathological abnormality or not, and a microscopic image block analysis result of each microscopic image block may present whether the sample cells have the pathological abnormality or not in real time. After all the microscopic image blocks are analyzed, an overall analysis result is obtained. When the sample has the pathological abnormality, the at least one microscopic image block analysis result of whether the sample cells have the pathological abnormality or not may be obtained when analysis is performed till a certain microscopic image block, but all the microscopic image blocks may be analyzed to obtain the overall analysis result during a practical operation, and in such case, a microscopic image block analysis result of a certain microscopic image block or at least one microscopic image block analysis result of some microscopic image blocks is consistent with the overall analysis result. However, when the sample does not have the pathological abnormality, the at least one microscopic image block analysis result of whether the sample cells have the pathological abnormality or not may be obtained after all the microscopic image blocks of the sample are analyzed, and in such case, a microscopic image block analysis result of each microscopic image block is consistent with the overall analysis result.

The display device is connected with the microscopic image analysis device and configured to output and display the at least one microscopic image block analysis result and the overall analysis result, or the at least one microscopic image block analysis result, or the overall analysis result to a user.

In an optional embodiment, the control element is configured to control the object stage to move in a stepping manner and control the camera to shoot once every time when the object stage moves to each of the multiple positions. A step length for moving in the stepping manner of the object stage is less than or equal to the width of the microscopic field shot by the camera.

In an optional embodiment, the camera is configured to shoot the sample row by row and transmit a shooting result in real time.

In an optional embodiment, the display device is a mobile terminal or a visual screen, implements data interaction with the image analysis device and outputs the analysis result in real time for observation of medical staff.

In another embodiment of the present invention, an image analysis device is further provided, which is configured to analyze a pathological microscopic image in real time and is characterized by including:

a microscopic image block acquisition component, establishing a data transmission connection with a microscopic image collection device and configured to acquire at least one microscopic image block corresponding to at least one position of a sample in real time at the same time when the microscopic image collection device sequentially shoots multiple positions of the sample; a microscopic image block analysis component, including a trained neural network model and configured to sequentially analyze the at least one microscopic image block corresponding to the at least one position to obtain at least one microscopic image block analysis result, each of the at least one microscopic image block analysis result including analysis results, corresponding to each microscopic image block, of whether sample cells have a pathological abnormality or not; an overall analysis component, configured to obtain an overall analysis result according to the at least one microscopic image block analysis result; and an output component, configured to output the at least one microscopic image block analysis result and the overall analysis result, or the at least one microscopic image block analysis result, or the overall analysis result.

In an optional embodiment, the image analysis device further includes an image stitching component, configured to stitch a microscopic image block corresponding to each position of the sample to obtain an overall microscopic sample image. The output component is further configured to output the overall microscopic sample image.

In an optional embodiment, the image analysis device further includes an image labeling component, configured to label a position of the pathological abnormality in the overall microscopic sample image according to the at least one microscopic image block analysis result. The output component is further configured to output the labeled overall microscopic sample image.

In an optional embodiment, the image analysis device further includes a microscopic image block analysis result determination component, configured to, every time after the at least one microscopic image block analysis result is obtained, determine whether the at least one microscopic image block analysis result meets a set condition, when the at least one microscopic image block analysis result meets the set condition, the output component is further configured to output the at least one microscopic image block analysis result, when the at least one microscopic image block analysis result does not meet the set condition the output component is further configured to output an overall analysis result after the overall analysis result is obtained.

In an optional embodiment, the neural network model is a Convolutional Neural Network (CNN) model. The trained neural network model is obtained by the following training steps: training data is obtained, the training data being a microscopic image of the sample and labeling information corresponding to the microscopic image; and the training data is input to a CNN model for training is performed to obtain the trained neural network model, the CNN model including at least one convolutional layer, at least one pooling layer and at least one batch normalization layer and a loss function for training the CNN model being:

$$\mathrm{loss}(x, class) = -x[class] + \log\left(\sum_j \exp(x[j])\right)$$

or

$$\mathrm{loss}(x, class) = weight[class]\left(-x[class] + \log\left(\sum_j \exp(x[j])\right)\right).$$

In another embodiment of the present invention, a real-time image analysis method is further provided, which is applied to real-time analysis of a pathological microscopic image and includes that: at least one microscopic image block corresponding to at least one position of a sample are acquired in real time at the same time of sequentially shooting multiple positions of the sample; the at least one microscopic image block is sequentially input to a trained neural network model for analyzing to obtain at least one microscopic image block analysis result, each of the at least one microscopic image block analysis result including an analysis result, corresponding to each microscopic image block, of whether sample cells have a pathological abnormality or not; an overall analysis result is obtained according to the at least one microscopic image block analysis result; and the overall analysis result is output.

In an optional embodiment, the method further includes that: every time after the at least one microscopic image block analysis result is obtained, whether the at least one microscopic image block analysis result meets a set condition is determined; when the at least one microscopic image block analysis result meets the set condition, the at least one microscopic image block analysis result is output; when the at least one microscopic image block analysis result does not meet the set condition, an overall analysis result is output after the overall analysis result is obtained.

In an optional embodiment, the set condition in the method is whether the abnormality exists or not or whether canceration exists or not.

In an optional embodiment, the sample in the method is any one of a human lung cell sample, a human cervical cell sample, a human mammary cell sample or a human thyroid cell sample.

In an optional embodiment, the sample in the method is a human cell or tissue acquired by puncturing or an endoscope.

In an optional embodiment, the human lung cell sample in the method is a human lung cell acquired by puncturing or a human lung cell acquired by a tracheoscope.

In an optional embodiment, the microscopic image block analysis result in the method includes that a qualitative examination result of canceration is negative or positive.

In an optional embodiment, the microscopic image block analysis result in the method may further be specifically subdivided into at least one of lung squamous carcinoma, a lung adenocarcinoma, small cell lung carcinoma, undefined non-small-cell lung carcinoma, another malignant lesion, granuloma and inflammation.

In an optional embodiment, the neural network model in the method is a CNN model. The trained neural network model is obtained by the following training steps: training data is obtained, the training data being the microscopic image of the sample and labeling information corresponding to the microscopic image; and the training data is input to a CNN model for training is performed to obtain a trained neural network model, the CNN model including at least one convolutional layer, at least one pooling layer and at least one batch normalization layer and a loss function for training the CNN model being:

$$\text{loss}(x, class) = -x[class] + \log\left(\sum_j \exp(x[j])\right)$$

or

$$\text{loss}(x, class) = weight[class]\left(-x[class] + \log\left(\sum_j \exp(x[j])\right)\right).$$

In an optional embodiment, the labeling information in the method includes at least two of lung squamous carcinoma, lung adenocarcinoma, small cell lung carcinoma, undefined non-small-cell lung carcinoma, another malignant lesion, no apparent abnormality, granuloma and inflammation.

In an optional embodiment, the method further includes that: a microscopic image block corresponding to each position of the sample is stitched to obtain an overall microscopic sample image.

In an optional embodiment, the method further includes that: a position of the pathological abnormality is labeled in the overall microscopic sample image according to the at least one microscopic image block analysis result.

In the application, the microscopic image block includes an un-preprocessed or preprocessed microscopic image block, and preprocessing includes a common preprocessing manner in this field such as normalization and resizing for the microscopic image block shot by the microscope.

In another embodiment of the present invention, a non-transitory computer-readable storage medium is further provided, which stores a computer-executable instruction. When the computer-executable instruction is executed, a computer executes real-time collection and real-time analysis of the abovementioned microscopic image block in the present invention and outputs an analysis result.

In another embodiment of the present invention, a neural network model training method for pathological microscopic image analysis is further provided, which includes that: training data is obtained, the training data being the microscopic image of a sample and labeling information corresponding to the microscopic image; and the training data is input to a CNN model for training is performed to obtain a trained neural network model, the CNN model including at least one convolutional layer, at least one pooling layer and at least one batch normalization layer and a loss function for training the CNN model being:

$$\text{loss}(x, class) = -x[class] + \log\left(\sum_j \exp(x[j])\right)$$

or

$$\text{loss}(x, class) = weight[class]\left(-x[class] + \log\left(\sum_j \exp(x[j])\right)\right).$$

In an optional embodiment, the neural network model training method further includes that the trained neural network model is tested by use of test data, specifically including that: the test data is obtained, the test data being a microscopic image, different from the training data, of the sample and labeling information corresponding to the microscopic image; the microscopic image in the test data is analyzed by use of the trained neural network model to obtain a microscopic image block test analysis result; and the microscopic image block test analysis result is compared with the labeling information in the test data to obtain a test comparison result.

According to at least some embodiments of the present invention, through the pathological microscopic image collection device, system and method adopting the technical solutions, the whole solution, compared with the related art, has the following advantages in image collection, analysis and displaying of pathological cells or tissues, specifically summarized as follows.

(1) Image collection and pathological analysis may be performed on cells or tissues in real time instead of image analysis processing after collection of all images in the related art, much time is bought for patients, and the technical problem, mentioned in Background, that relatively long time is required by pathological analysis in an operating process is solved.

(2) With adoption of a neural network training method, the pathological analysis accuracy in the present invention is remarkably improved, and a more accurate pathological result may be obtained by analysis with a trained neural network.

(3) The training method adopted in the present invention is quick in response, and thus the processing speed for pathological analysis, compared with that of a conventional method, is greatly improved.

**Brief Description of the Drawings**

Fig. 1 is a schematic diagram of a real-time pathological microscopic image collection and analysis system according to an embodiment of the present invention.

Fig. 2 is a structural block diagram of an image analysis device according to an embodiment of the present invention.

Fig. 3 is a flowchart of a real-time image analysis method according to an embodiment of the present invention.

Fig. 4 is a flowchart of a neural network model training method for pathological microscopic image analysis according to an embodiment of the present invention.

Fig. 5 is a schematic diagram of a CNN model according to an embodiment of the present invention.

Fig. 6 is a schematic diagram of a microscopic image of lung cells without any apparent abnormality according to an exemplary embodiment of the present invention.

Fig. 7 is a schematic diagram of a microscopic image lung cells with adenocarcinoma according to an exemplary embodiment of the present invention.

**Detailed Description**

For describing the objective of the present invention, implementation of the technical solutions and the advantages of the present invention compared with the related art better, the present invention will further specifically be elaborated and described in combination with the shown drawings and examples of different embodiments. It should be understood that the described or exemplary specific embodiments are adopted to explain or conveniently understand the overall inventive concept of the present invention but should not be understood as limits to the scope of protection of the claims of the present invention. All contents in the inventive concept and core of the present invention shall fall within the scope of protection of the present invention, and particularly equivalent replacements or specific transformations made based on the inventive idea or subject of the present invention fall within the scope of protection of the present invention.

According to at least some embodiments of the present invention, sample cells at a bronchial or pulmonary lesion are mainly collected through a transbronchial lung biopsy and further analyzed and processed, thereby aiding in disease diagnosis.

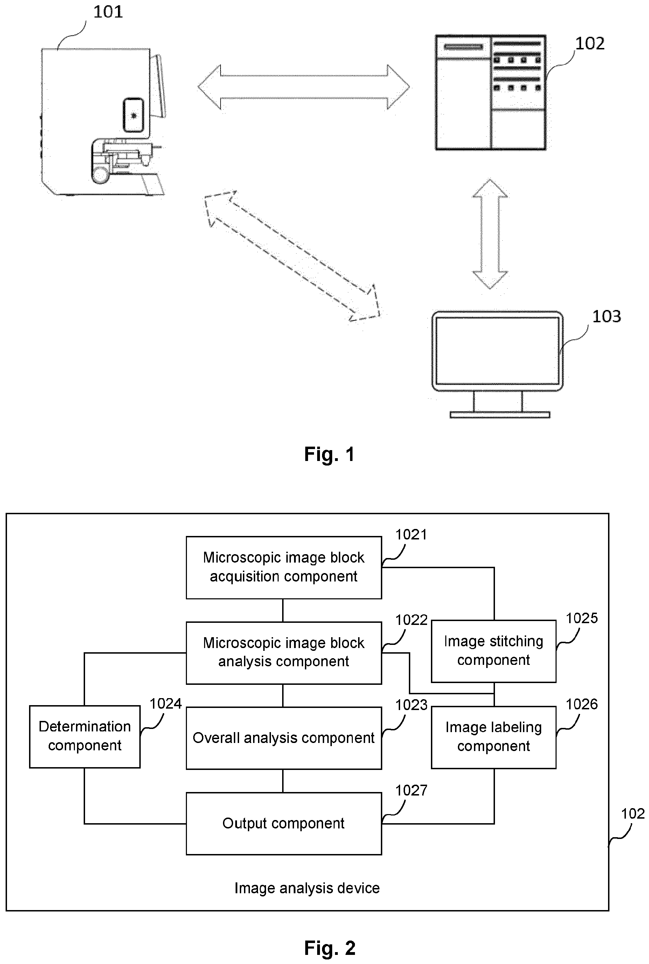

A method provided in some embodiments of the present invention may be applied to a system shown in Fig. 1. An image analysis device in the system may be a computer device, including a processor and memory connected through a system bus. A computer program is stored in the memory. The processor, when executing the computer program, may execute the steps of the following method embodiment. In an optional embodiment, the computer device may further include a network interface, a display screen and an input device. The processor of the computer device is configured to provide calculation and control capabilities. The memory of the computer device includes a non-transitory storage medium and an internal memory. The non-transitory storage medium stores an operating system and the computer program. The internal memory provides a running environment for the operating system and computer program in the non-transitory storage medium. The network interface of the computer device is configured for communication with an external terminal through a network connection. In an optional embodiment, the computer device may be a server, may also be a personal computer, may also be a personal digital assistant, may also be another terminal device such as a tablet computer and a mobile phone, and may also be a cloud or remote server. A specific form of the computer device is not limited in the embodiments of the application.

The technical solutions of the present invention and how to solve the abovementioned technical problems through the technical solutions of the present invention will be described below with specific embodiments in detail. The following specific embodiments may be mutually combined, and the same or similar processes may not be elaborated in some embodiments.

Embodiment One

This embodiment provides a real-time pathological microscopic image collection and analysis system and a real-time image analysis method.

As shown in Fig. 1, the real-time pathological microscopic image collection and analysis system includes a microscopic image collection device 101, an image analysis device 102 and a display device 103.

The microscopic image collection device 101 includes an object stage, a camera and a control element. The object stage is configured to carry a sample. The sample may be a human cell or tissue sample. For example, the sample may be a cell or tissue extracted from any part of a human body such as the lung, thyroid and breast of the human body and configured for pathological microscopic diagnosis. An acquisition method for the human cell or tissue sample may be acquisition by a puncturing operation and may also be acquisition through an endoscope or acquisition by other medical means. The sample is usually made into a microscopic slide and placed on the object stage. In some cases, it is necessary to perform processing such as straining on the sample slide to distinguish cells more clearly. The camera is configured to shoot the sample to obtain a microscopic image. In some embodiments, the camera is connected with an eyepiece of a microscope and configured to shoot a microscopically magnified sample image. The control element is configured to control relative movement of the object stage and the camera and control the camera to sequentially shoot multiple positions of the sample to obtain microscopic image blocks. In an optional embodiment, the object stage is movable in two mutually perpendicular directions X and Y on a horizontal plane, and the object stage is driven by a driving device to move. For example, the driving device may be a stepper motor. The control element may control the object stage to move in a stepping manner and control the camera to shoot once every time when the object stage moves to each of the plurality of positions. A step length for moving in the stepping manner of the object stage is less than or equal to a width of a microscopic field shot by the camera. After a row of the sample is shot in the direction C, the object stage is controlled to move by the step length in the direction Y, and a next row is continued to be shot.

The image analysis device 102 is configured to acquire at least one microscopic image block in real time, input the at least one microscopic image block to a trained neural network model for analyzing to obtain at least one microscopic image block analysis result, each of the at least one image block analysis result including an analysis result, corresponding to each microscopic image block, of whether sample cells have a pathological abnormality or not, and after all the collected microscopic image blocks are analyzed, obtain an overall analysis result. The image analysis device 102 is connected with the microscopic image collection device 101 and may receive and analyze image data transmitted by the microscopic image collection device 101 in real time. A connection manner for the image analysis device 102 and the microscopic image collection device 101 should include any connection manner in which data may be transmitted in real time, for example, wired connection and wireless connection (for example, Wireless Fidelity (WIFI) connection and Bluetooth connection), and may also be internal connection of different components in an integrated machine. For another example, the connection manner for the image analysis device 102 and the microscopic image collection device 101 may also be cloud connection. For example, the microscopic image collection device 101 is arranged on a diagnosis and treatment site of a hospital, while the image analysis device 102 may be a cloud server, and through the general Internet or a 5th-Generation (5G) network, the microscopic image collection device 101 may transmit the collected image block to the image analysis device 102 in real time for analysis and diagnosis. The microscopic image collection device 101 should transmit the at least one microscopic image block to the image analysis device 102 in real time. Here, real-time transmission should be understood as that the microscopic image collection device 101 has started transmitting the image data to the image analysis device 102 in a sample shooting process. In an example, every time when shooting a microscopic image block, the microscopic image collection device 101 transmits this microscopic image block to the image analysis device 102 immediately. In another example, every time when shooting a microscopic image block of a row in the direction X, the microscopic image collection device 101 transmits image data of the shot row to the image analysis device 102.

The display device 103 is configured to output and display the at least one microscopic image block analysis result and the overall analysis result, or the at least one microscopic image block analysis result, or the overall analysis result to a user. It can be understood that the display device 103 is connected with the image analysis device 102 and a connection manner may be any connection capable of transmitting data such as wired connection and wireless connection. In one embodiment, the display device 103 and the image analysis device 102 may be two components in an integrated machine. In another embodiment, the display device 103 may be connected with the image analysis device 102 in WIFI, Bluetooth and cloud connection manners, etc.

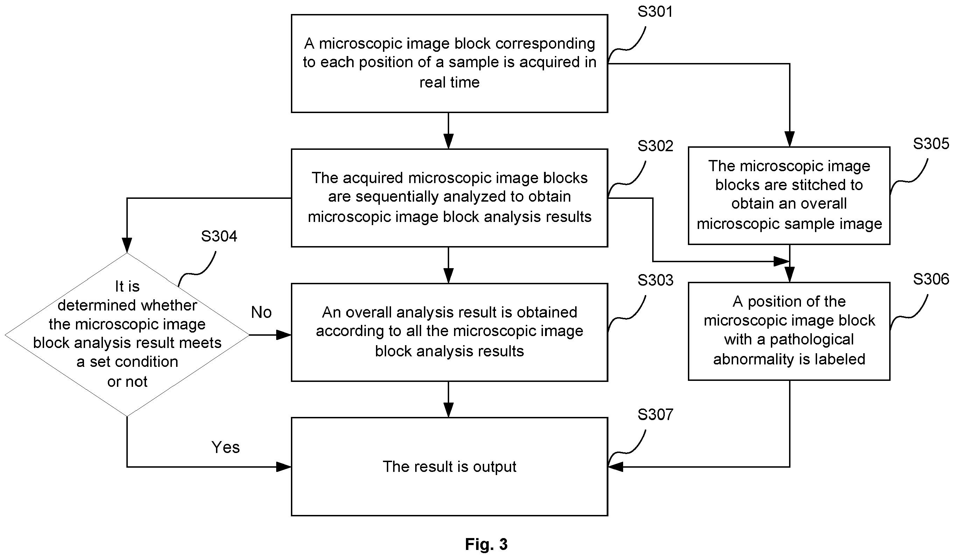

Functions of each component of the image analysis device 102 and specific steps of the real-time image analysis method will specifically be described below in combination with Fig. 2 and Fig. 3. A non-transitory computer-readable storage medium is also provided. The non-transitory computer-readable medium stores a computer-executable instruction, and when the computer-executable instruction is executed, a computer executes the real-time image analysis method recorded in the present invention.

As shown in Fig. 3, in an embodiment, a real-time image analysis method for real-time pathological microscopic image analysis includes the following basic steps.

In step S301, a microscopic image block corresponding to each position of a sample is acquired in real time.

In step S302, the acquired microscopic image blocks are sequentially analyzed to obtain microscopic image block analysis results.

In step S303, an overall analysis result is obtained according to all the microscopic image block analysis results.

In step S307, the overall analysis result is output. The method is specifically as follows.

In step S301, a microscopic image block corresponding to each position of a sample is acquired in real time. The step may be executed by a microscopic image block acquisition component 1021 in the image analysis device 102.

In step S302, the acquired microscopic image blocks are sequentially analyzed to obtain microscopic image block analysis results. The step may be executed by a microscopic image block analysis component 1022 in the image analysis device 102. In an optional embodiment, the microscopic image block analysis component 1022 includes a trained neural network model and is configured to sequentially analyze the microscopic image blocks to obtain the microscopic image block analysis results. In an optional embodiment, the microscopic image block analysis result includes an analysis result, corresponding to each microscopic image block, of whether sample cells have a pathological abnormality or not. The analysis result of the pathological abnormality may be an analysis result of a specific pathological abnormality, for example, whether canceration exists or not (canceration negative or canceration positive) and whether there is an inflammation or not, and may also be classification of multiple pathological conditions, for example, diagnosis of lung cells may be divided into multiple classes (divided into no abnormality, inflammation, squamous carcinoma, adenocarcinoma, small cell carcinoma and the like).

It can be understood that the trained neural network model in the microscopic image block analysis component 1022 may be a deep learning classification network model, deep learning object detection network model or deep learning segmentation network model for image processing. Taking the deep learning classification network model as an example, the model may be a neural network model obtained by adaptive parameter regulation and training with training data based on a deep learning classification network model such as VGG, Inception, ResNet, a Wide Residual Network (WRN) and SqueezeNet. Taking the deep learning object detection network model as an example, the model may be a neural network model obtained by adaptive parameter regulation and training with training data based on a deep learning target detection network model such as a Recurrent Convolutional Neural Network (RCNN), a Spatial Pyramid Pooling (SPP) Net, a Fast RCNN and a Feature Pyramid Network (FPN). It can be understood by those skilled in the art that, if the deep learning classification network model is trained, the corresponding training data for training the model should include an image and classification labeling information of the image, and the classification labeling information may be a binary classification label (the label is positive or negative, for example, for diagnosis specifically for lung adenocarcinoma, the label is positive when lung adenocarcinoma exists and is negative when lung adenocarcinoma does not exist) and may also be a multi-classification label for multiple pathological conditions (for example, classes for the lung cells include no abnormality, inflammation, squamous carcinoma, adenocarcinoma, small cell carcinoma and the like). When the deep learning object detection network model is trained, the corresponding training data for training the model should include at least one image and object labeling information corresponding to each of the at least one image.

In step S303, an overall analysis result is obtained according to all the microscopic image block analysis results. The step may be executed by an overall analysis component 1023 in the image analysis device 102. In this step, the microscopic image block analysis results obtained in S302 may be simply summarized, or further analysis processing may be performed on the basis of summarizing the results obtained in S302. For example, when the microscopic image block analysis result of each image block, obtained by performing multi-classification processing on the microscopic image block of the lung cell sample in step S302, includes multiple lesions (for example, including inflammation and canceration), summarization and further analysis may be performed on multiple results in step S303. For example, further analysis may be counting the microscopic image blocks where canceration exists or counting cancerous cells to analyze a canceration degree and the like.

In step S307, the overall analysis result is output. The step may be executed by an output component 1027 in the image analysis device 102. The output component 1027 may directly process the overall analysis result obtained in the abovementioned steps (S302 and S303, etc.) into a signal to be output and transmit the signal to the display device 103 for displaying to a user.

S301, S302, S303 and S307 are one exemplary implementation mode. In an optional embodiment, as shown in Fig. 2 and Fig. 3, other varied implementation modes may also be included.

Since an implementation scenario of the technical solution is usually a site of operating a patient, for further obtaining an analysis and diagnosis result rapidly, as an embodiment, the image analysis device 102 further includes a Determination component 1024, configured to execute step S304 to determine whether the at least one microscopic image block analysis result meets a set condition or not, and when a determination result is Yes, step S307 is immediately executed to process the at least one microscopic image block analysis result into the signal to be output and transmit the signal to the display device 103 for displaying to the user. It can be understood that the set condition in the determination step may be set by a system and may also be set by the user. For example, the set condition may include whether canceration exists or not, whether there is an inflammation or not, whether there is another pathological abnormality or not and the like.

In an optional embodiment, the image analysis device 102 further includes an image stitching component 1025, configured to execute step S305 to acquire the microscopic image blocks obtained in step S301 and stitch the microscopic image blocks to obtain an overall microscopic sample image. It can be understood that, after the step is completed, step S307 may be directly executed to process the overall microscopic image into the signal to be output and transmit the signal to the display device 103 for displaying to the user.

In an optional embodiment, the image analysis device 102 further includes an image labeling component 1026, configured to execute step S306 to label a position of the pathological abnormality in the overall microscopic image obtained in step S305. In one example, a labeling manner for the position of the pathological abnormality is labeling the cells with the pathological abnormality in the overall microscopic image. In another example, the labeling manner for the position of the pathological abnormality is labeling the microscopic image block with the pathological abnormality in the overall microscopic image. After step S306 is completed, step S307 is executed to process the labeled overall microscopic sample image into the signal to be output and transmit the signal to the display device 103 for displaying to the user.

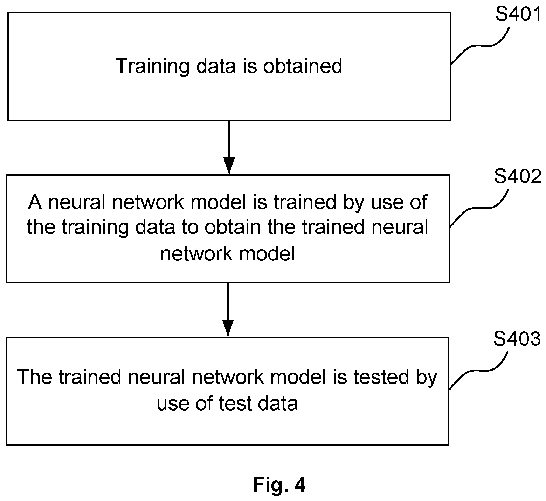

A training method for the neural network model used in step S302 will be described with an example below in combination with Fig. 4. As shown in Fig. 4, the trained neural network model used in step S302 is obtained by the following training method.

In step S401, training data is obtained. The training data for the neural network model includes a microscopic image of the sample and labeling information corresponding to the microscopic image. For example, the sample may be a cell or tissue of a part of a human body such as the lung, the thyroid and the breast. The microscopic image of the sample may be a microscopic image block and may also be an overall microscopic sample image obtained by stitching. The labeling information may be a label made to the microscopic image by a pathologist or an expert mastering professional knowledge. Different manners may be adopted for the labeling information. For example, the labeling information may be a simple class of each image in an image set, such as, whether there is a lesion or not and whether canceration exists or not, or the labeling information may be a multi-classification label, such as, each image in the image set is subdivided into squamous carcinoma, adenocarcinoma, small cell carcinoma, inflammation and no abnormality, or the labeling information may also be an outline of a position of a lesion, such as, the cell that the lesion occurs to in the image is outlined. It can be understood that different labeling manners may be applied to training of different types of neural network models (for example, a deep learning classification network and a deep learning object detection network).

In step S402, a neural network model is trained by use of the training data to obtain the trained neural network model. The neural network model is trained according to the training data obtained in step S401 to obtain the trained neural network model. It can be understood that the step may be implemented based on multiple neural network models. For example, training may be performed based on the deep learning classification network (for example, a CNN, VGG, Inception, ResNet, a WRN and SqueezeNet) by taking a microscopic image labeled with classification information as training data, or training may be performed based on the deep learning object detection network (for example, an RCNN, an SPP Net, a Fast RCNN and an FPN) by taking a microscopic image labeled with lesion position information as training data. It can be understood by those skilled in the art that a neural network model obtained by training based on the deep learning classification network may be configured to perform classification processing on a microscopic image block to be analyzed to obtain a classification result as an image block analysis result and a neural network model obtained by training based on the deep learning object detection network may be configured to perform object detection on the microscopic image block to be analyzed to obtain an object detection result as the image block analysis result.

In an optional embodiment, a microscopic image labeled with classification information is input to a CNN model as training data, and training is performed to obtain a trained CNN model. The CNN model includes at least one convolutional layer, at least one pooling layer and at least one batch normalization layer, and a loss function for training the CNN model is:

$$\text{loss}(x, class) = -x[class] + \log \left( \sum_j \exp(x[j]) \right),$$

or

$$\text{loss}(x, class) = weight[class] \left( -x[class] + \log \left( \sum_{j} \exp(x[j]) \right) \right).$$

And, loss represents the loss function, weight represents a weight, x represents a predicted probability, class represents a corresponding class, and j represents serial numbers of all classes. For weighted cross entropy, proportions of training sets corresponding to different disease entities are different, adopting the weighted cross entropy as the loss function for training may ensure higher classification accuracy of the trained neural network model.

After step S402 is completed, the trained neural network model for step S302 is obtained. For ensuring the quality of the trained neural network model, before the model is put into use, the model may be tested through the following step.

In step S403, the trained neural network model is tested by use of test data. The test step specifically includes the following steps. In step S4031, the test data is obtained. The test data is a microscopic image, different from the training data, of the sample and labeling information corresponding to the microscopic image. A specific method for obtaining the test data may refer to the method in S401 and will not be elaborated herein. In step S4032, the microscopic image in the test data is analyzed by use of the trained neural network model to obtain a microscopic image block test analysis result. In step S4033, the microscopic image block test analysis result is compared with the labeling information in the test data to obtain a test comparison result.

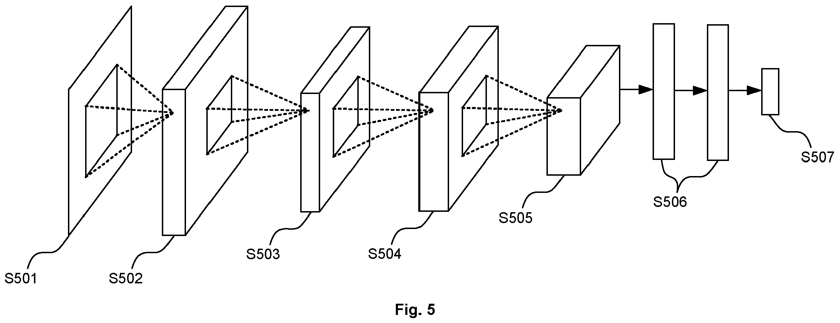

The solution recorded above is the typical embodiment of the present invention. For describing a method for analyzing the microscopic image block by use of the trained neural network model in step S302 in the embodiment more completely, descriptions will further be made below with a CNN as an example in combination with Fig. 5.

As shown in Fig. 5, a CNN model for analyzing a microscopic image of a sample includes an input layer 501, a convolutional layer 502, a pooling layer 503, a convolutional layer 504, a pooling layer 505, a fully connected layer 506 and an output layer 507. The input layer 501 executes inputting of an image. The image is the microscopic image block of the sample. The image may be considered as a two-dimensional array consisting of many pixels, and each pixel has a respective pixel value. The convolutional layers 502 and 504 are configured to perform feature extraction on the input image. In a CNN, there may be at least one convolutional layer. For an 8x8 input image, the convolutional layer calculates a 6x6 feature map by use of a 3x3 convolution kernel. During convolution calculation, a size of an output feature map is smaller than an original image, and when a larger convolution kernel is adopted, the obtained feature map is smaller. For an nxm convolution kernel, the size of the input image may be reduced by $(n-1) \times (m-1)$. Therefore, when a 5x5 convolution kernel is adopted, the feature map is 4x4. In many cases, the feature map is required to be as large as the original image and then the feature map is required to be filled, usually filled with 0. When the size of the original image is 8x8 and the kernel is 5x5, the original image is required to be filled to 12x12 at first by adding four additional rows and columns, two rows/columns on each side. For reducing the calculation burden and increasing the calculation speed, a pooling layer may be generated for the convolutional layer to down-sample the feature map to generate a feature map with a smaller size. The fully connected layer 506 is configured to map a feature obtained by down-sampling of the pooling layer 505 to a sample labeling space. Finally, the classification result is output through the output layer 507.

Embodiment Two (for lung tissues or cells)

A sample ROSE technology, commonly used in this field at present, refers to that a cell pathologist rapidly examines sample cells on site and evaluates the quality of a fine needle aspiration smear and a bioptic imprint. An application scenario of ROSE is usually a transbronchial bioptic imprint collected by transbronchial biopsy and a fine needle aspiration smear collected by fine needle aspiration.

In the embodiment of the present invention, a pathological microscopic image of cells is shot by a digital microscope: the transbronchial bioptic imprint or the fine needle aspiration smear is scanned by the microscope. A magnification factor of the microscope is as follows: a magnification factor of an objective lens may be 10, 20 and 100, a magnification factor of an eyepiece may be 10, and the objective lens and the eyepiece may be matched to achieve a magnification factor of 100 to 1,000. In a scanning shooting process, a size of a lung cell sample slide is usually several centimeters, while a field shot by the microscope at one time is very small. A complete large image consists of hundreds of and even thousands of microscopic image blocks (correlated with a shot range of the slide and the magnification factor) and includes hundreds of million pixels. In this case, the size of the slide is 76*26mm (the slide is not completely shot and the shot range of the slide is adjustable). Since a middle part of the slide is usually smeared with the sample, there is usually no sample on an edge of the shot range of the slide. However, during a practical operation, a slide part on an edge of the sample may still be shot to ensure that the shot range of the sample is complete. An object stage, carrying the slide, of the microscope is controlled by a control element to move to scan and shoot the slide row by row in a stepping manner. Shooting of the sample may be completed by shooting for 100 times, totally 10 rows being shot with each row shot for 10 times.

In the scanning process, a camera shoots once to generate a picture of a microscopic image block, and every time when a picture of a microscopic image block is generated, the picture is immediately transmitted to a computer device for analysis. A size of the picture of the microscopic image block is 1,936*1,216 pixels (after the sample is magnified by the microscope and shot by the camera, an optical signal is converted into a digital signal, each pixel being 0.24 micron wide). A microscopic image block acquisition component of the computer device, after receiving the picture of the microscopic image block, performs normalization and resizing and sends the processed picture to a microscopic image analysis component for processing and analysis through a trained CNN. Binary classification, for determining whether canceration exists or not, may be performed on these cells to obtain a negative or positive conclusion. Or, in an implementation process, a specific class of the picture in lung squamous carcinoma, lung adenocarcinoma, small cell lung carcinoma, undefined non-small-cell lung carcinoma, another malignant lesion, no apparent abnormality, granuloma and inflammation may be determined. In the embodiment, multi-classification (eight classes) processing is performed on the lung cells by use of a deep learning classification neural network. Since a middle position of the slide is smeared with the cells, no sample is shot when the first two rows are shot, the sample is shot (shot for the 25th time) from a middle position of the third row, but the microscopic image block analysis component does not obtain an analysis result indicating existence of an abnormality, and when shooting for the 26th time is completed, a corresponding image block is analyzed to find adenocarcinoma cells. Shooting time in each stepping process is averagely 0.5 second, a process of transmission to the trained CNN for real-time analysis may be delayed for 0.5 second, total time for completing shooting for 26 times is 13 seconds, and time for completing analyzing the image block is 0.5 second, so that the diseased cells are found by analysis for 13.5 seconds, and a diagnosis result may be output immediately. That is, totally 13.5 seconds are required from starting of sample shooting to obtaining of the adenocarcinoma cells in the sample. Therefore, a timely reference is provided for rapid determination of a disease condition by a doctor that performs an operation.

In the embodiment of the present invention, after all the 100 pictures are analyzed, the output result, i.e., an overall analysis result, is output. Total time does not exceed 1 minute.

According to the method and the device, ROSE may be implemented in real time in an operating room, or a respiratory physician may be assisted in ROSE, to greatly accelerate ROSE and reduce the risk of the patient waiting for a ROSE result on an operating table.

A training process for the CNN for cell pathology analysis and processing in the embodiment of the present invention is as follows.

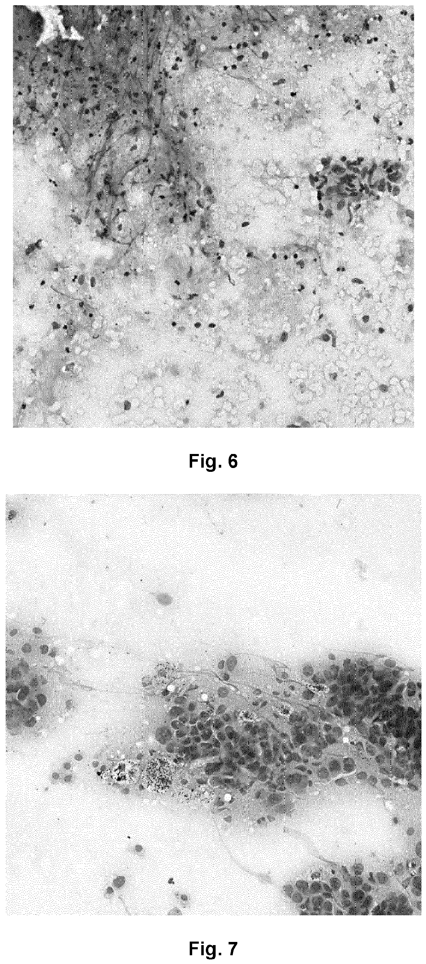

(1) After collected picture information is obtained, pictures obtained by scanning a transbronchial bioptic imprint and fine needle aspiration smear through a microscope are labeled and divided into totally eight classes, i.e., no apparent abnormality (as shown in Fig. 6), lung squamous carcinoma, lung adenocarcinoma (shown in Fig. 7), small cell lung carcinoma, undefined non-small-cell lung carcinoma, another malignant lesion, granuloma and inflammation respectively.

(2) 70% of data is adopted for training and input to a CNN including at least one convolutional layer, at least one pooling layer and at least one batch normalization layer. A loss function is a cross entropy or weighted cross entropy loss function:

$$\text{loss}(x, class) = -x[class] + \log\left(\sum_j \exp(x[j])\right)$$

or

$$\text{loss}(x, class) = weight[class]\left(-x[class] + \log\left(\sum_j \exp(x[j])\right)\right).$$

And loss represents the loss function, weight represents a weight, x represents a predicted probability, class represents a corresponding class, and j represents serial numbers of all classes. For the weighted cross entropy, proportions of training sets corresponding to different disease entities are different, adopting the weighted cross entropy as the loss function for training may ensure higher classification accuracy of a trained neural network model.

(3) 20% of the data is adopted to calculate a loss value after iterative updating of a parameter in training to determine the quality of the model, and when the loss value decreases to a relatively small value and does not continue decreasing, model training is completed.

(4) 10% of the data is adopted to test the trained model. This data may not participate in model fitting as well as calculation of the loss value, namely this data does not participate in the whole training process, so that a test result is more objective. The accuracy of the test result is the accuracy, expected to be achieved by the model, of labeled data.

The trained CNN is configured to analyze a microscopic image of a sample in the embodiment. As shown in Fig. 5, the CNN model includes an input layer 501, a convolutional layer 502, a pooling layer 503, a convolutional layer 504, a pooling layer 505, a fully connected layer 506 and an output layer 507. Details about a parameter configuration of each layer refer to Table 1.

Table 1 Parameter Configuration of CNN Model

| Layer name | Kernel size | Step length | Output size | Number of feature maps |
|---|---|---|---|---|
| Input layer 501 | | | 224*224 | 3 |
| Convolutional layer 502 | 7*7 | 2 | 112*112 | 64 |
| Pooling layer 503 | 3*3 | 2 | 56*56 | 64 |
| Convolutional layer 504 | $\begin{bmatrix} 1 & * & 1 \\ 3 & * & 3 \end{bmatrix}*6$ | 1 | 56*56 | 128 |
| Pooling layer 505 | 2*2 | 2 | 28*28 | 128 |
| Fully connected layer 506 | | | 1*1*1 | 2 |
| Output layer | | | 1*1* | 2 |

The input layer 501 is configured to input a microscopic image block collected by the microscopic image collection device to the CNN. An original size of the microscopic image block is 1,936*1,216 pixels. For adaptation to the input of the CNN, the original image is down-sampled to obtain a 224*224 feature map. A microscopic image block is usually a color image represented by three color values Red Green Blue (RGB). Therefore, three 224*224 feature maps are generated.

The convolutional layer 502 is configured to perform feature extraction on the input microscopic image block. In one example, for the three 224*224 input feature maps, the convolutional layer 502 calculates 64 112*112 feature maps by use of a 7*7 convolution kernel.

For reducing the calculation burden and increasing the calculation speed, the pooling layer 503 may be generated for the convolutional layer 502 to down-sample the feature map to generate a feature map with a smaller size. In one example, the pooling layer 503 has a 3*3 kernel and performs down-sampling according to the 64 112*112 feature maps generated by the convolutional layer 502 to obtain 64 56*56 feature maps.

The convolutional layer 504 includes a convolutional layer with a 1*1 convolution kernel and another convolutional layer with a 3*3 convolution kernel, which are connected in series, and 6 cycles are executed on the layer to obtain 128 56*56 feature maps.

The pooling layer 505 is connected with the convolutional layer 504. The pooling layer 505 has a 2*2 kernel and performs down-sampling according to the 56*56 feature maps generated by the convolutional layer 504 to obtain 28*28 feature maps.

The fully connected layer 506 is configured to map features obtained by down-sampling of the pooling layer 505 to a sample labeling space. Finally, the classification result is output through the output layer 507. The classification result is a determination result of whether the microscopic image block has a pathological abnormality or not.

Embodiment Three (for breast tissues or cells)

In the embodiment, binary classification, namely positive and negative, is performed on the breast cells by use of a deep learning classification neural network, namely a diagnosis result is negative or positive. A breast tissue or breast cell slide of a human body is collected, the slide is placed on the object stage, the same system in embodiment one is selected, and a scanning process is the same as that in embodiment two. The difference is that: the microscope scans the slide including a sample row by row, and shooting of the sample is completed by shooting for 144 times, totally 12 rows being shot with each row shot for 12 times. When shooting for the 52nd time is completed, a corresponding image block is analyzed to find cancerous cells. Shooting time in each stepping process is averagely 0.5 second, a process of transmission to a trained CNN for real-time analysis may be delayed for 0.5 second, total time for completing shooting for 52 times is 26 seconds, and time for completing analyzing the image block is 0.5 second, so that the cancerous cells are found by analysis for 26.5 seconds, and a diagnosis result indicating canceration positive may be output immediately. That is, totally 26.5 seconds are required from starting of sample shooting to obtaining of the cancerous cells in the sample. Therefore, a timely reference is provided for rapid determination of a disease condition by a doctor that performs an operation.

In the embodiment of the present invention, after all the 144 pictures are analyzed, the output result, i.e., an overall analysis result, is output. Total time does not exceed 2 minutes.

The same training method in embodiment 2 is adopted for a training process for the CNN for cell pathology, and a weighted cross entropy loss function may be selected according to breast traits to obtain a more accurate result. The following specific steps are included.

(1) After collected picture information is obtained, pictures obtained by scanning the breast cell or tissue slide through the microscope is labeled and divided into totally two classes, namely canceration negative (canceration does not exist) and canceration positive (canceration exists).

(2) 70% of data is adopted for training and input to a CNN including at least one convolutional layer, at least one pooling layer and at least one batch normalization layer. A loss function is a cross entropy or weighted cross entropy loss function:

$$\mathrm{loss}(x, class) = weight[class]\left(-x[class] + \log\left(\sum_j \exp(x[j])\right)\right).$$

And loss represents the loss function, weight represents a weight, x represents a predicted probability, class represents a corresponding class, and j represents serial numbers of all classes. For the weighted cross entropy, proportions of training sets corresponding to different disease entities are different, adopting the weighted cross entropy as the loss function for training may ensure higher classification accuracy of a trained neural network model.

(3) 20% of the data is adopted to calculate a loss value after iterative updating of a parameter in training to determine the quality of the model, and when the loss value decreases to a relatively small value and does not continue decreasing, model training is completed.

(4) 10% of the data is adopted to test the trained model. This data may not participate in model fitting as well as calculation of the loss value, namely this data does not participate in the whole training process, so that a test result is more objective. The accuracy of the test result is the accuracy, expected to be achieved by the model, of labeled data.

The trained CNN is configured to analyze the microscopic image block of the sample in the embodiment. Specific contents are the same as those in embodiment two and will not be elaborated.

Embodiment Four (for thyroid tissues or cells)

A thyroid tissue or cell slide of a human body is collected, the thyroid tissue or cell slide is placed on the object stage, the same system in embodiment one is selected, and a scanning process is the same as that in embodiment two. The difference is that: the microscope scans the slide including a sample row by row, and shooting of the sample is completed by shooting for 400 times, totally 20 rows being shot with each row shot for 20 times. Every time when a row is shot, image data of the row is transmitted to the image analysis device for analysis. When the third row is shot, an image block of the row is analyzed to find thyroid papillary carcinoma cells. Shooting time for each row is 10 seconds, a process of transmission to a trained CNN for real-time analysis may be delayed for 5 second, total time for completing shooting the third row is 30 seconds, and time for completing analyzing the image block of the row is 5 second, so that the cancerous cells are found by analysis for 35 seconds, and a diagnosis result indicating thyroid papillary carcinoma may be output immediately. That is, totally 35 seconds are required from starting of sample shooting to obtaining of the cancerous cells in the sample. Therefore, a timely reference is provided for rapid determination of a disease condition by a doctor that performs an operation. In the embodiment of the present invention, after 20 rows are all analyzed, the output result, i.e., an overall analysis result, is output. Total time does not exceed 4 minutes.

The same training method in embodiment two is adopted for a training process for the CNN for cell pathology, and a weighted cross entropy loss function may be selected according to thyroid traits to obtain a more accurate result. The following specific steps are included.

(1) After collected picture information is obtained, pictures obtained by scanning the thyroid cell or tissue slide through the microscope are labeled and divided into totally five classes, i.e., no abnormality, thyroid papillary carcinoma, thyroid follicular carcinoma, thyroid medullary carcinoma and anaplastic thyroid carcinoma respectively.
(2) 70% of data is adopted for training and input to a CNN including at least one convolutional layer, at least one pooling layer and at least one batch normalization layer. A loss function is a cross entropy or weighted cross entropy loss function:

$$\text{loss}(x, class) = -x[class] + \log\left(\sum_j \exp(x[j])\right).$$

And loss represents the loss function, weight represents a weight, x represents a predicted probability, class represents a corresponding class, and j represents serial numbers of all classes. For the weighted cross entropy, proportions of training sets corresponding to different disease entities are different, adopting the weighted cross entropy as the loss function for training may ensure higher classification accuracy of a trained neural network model.
(3) 20% of the data is adopted to calculate a loss value after iterative updating of a parameter in training to determine the quality of the model, and when the loss value decreases to a relatively small value and does not continue decreasing, model training is completed.
(4) 10% of the data is adopted to test the trained model. This data may not participate in model fitting as well as calculation of the loss value, namely this data does not participate in the whole training process, so that a test result is more objective. The accuracy of the test result is the accuracy, expected to be achieved by the model, of labeled data.

The trained CNN is configured to analyze the microscopic image block of the sample in the embodiment. Specific contents are the same as those in embodiment two and will not be elaborated.
The whole set of device system of the present invention may be placed on an operating site. After a cell sample of a patient is obtained by biopsy or puncturing, examination and result outputting for timely diagnosis may be implemented rapidly, so that the efficiency is greatly improved. Another improvement of the present invention is that: a cell pathology diagnosis is usually required to be made by an experienced doctor, the accuracy of a diagnosis made by an inexperienced doctor cannot be ensured, and there is likely a careless omission during diagnosis of a doctor because the size of a microscopic image is quite large; and diagnosing through the trained neural network model may ensure the diagnosis accuracy. Compared with the related art, the present invention has the following advantages, summarized as follows.

(1) Image collection and pathological analysis may be performed on cells or tissues in real time instead of image analysis processing after collection of all images in the related art, much time is bought for patients, and the technical problem, mentioned in Background, that relatively long time is required by pathological analysis in an operating process is solved.
(2) With adoption of a neural network training method, the pathological analysis accuracy in the present invention is remarkably improved. By contrast, a more accurate pathological result may be obtained by analysis with a trained neural network.
(3) The training method adopted in the present invention is quick in response, and thus the processing speed for pathological analysis, compared with that of a conventional method, is greatly improved.

The above are exemplary embodiments of the present invention but not intended to limit the present invention. All technical solutions within the spirit of the present invention shall fall within the scope of protection of the present invention. All transformations and equivalent replacements made based on the technical solutions in the spirit of the present invention shall fall within the scope of protection of the present invention.

**Claims**

**1.** A real-time pathological microscopic image collection and analysis system, configured to perform microscopic image collection and real-time analysis and diagnosis on a sample, the sample comprising at least one human cell or tissue, the system comprising:

(1) a microscopic image collection device, comprising:

an object stage configured to carry the sample;
a camera configured to shoot the sample to obtain a microscopic image; and
a control element, configured to control relative movement of the object stage and the camera and control the camera to sequentially shoot a plurality of positions of the sample to obtain microscopic image blocks;

(2) an image analysis device, connected with the microscopic image collection device and configured to acquire at least one microscopic image block corresponding to at least one position of the sample in real time at the same time when the microscopic image collection device sequentially shoots the plurality of positions of the sample, input the at least one microscopic image block to a trained neural network model in real time for analyzing to obtain at least one microscopic image block analysis result, each of the at least one microscopic image block analysis result comprising an analysis result, corresponding to each microscopic image block, of whether sample cells have a pathological abnormality or not, and after all the microscopic image blocks are analyzed, obtain an overall analysis result; and
(3) a display device, connected with the image analysis device and configured to output and display the at least one microscopic image block analysis result and the overall analysis result, or the at least one microscopic image block analysis result, or the overall analysis result to a user.

2. The real-time pathological microscopic image collection and analysis system as claimed in claim 1, wherein the control element is configured to control the object stage to move in a stepping manner and control the camera to shoot once every time when the object stage moves to each of the plurality of positions, and a step length for moving in the stepping manner of the object stage is less than or equal to a width of a microscopic field shot by the camera.

3. The real-time pathological microscopic image collection and analysis system as claimed in claim 1, wherein the camera is configured to shoot the sample row by row and transmit a shooting result in real time.

4. The real-time pathological microscopic image collection and analysis system as claimed in claim 1, wherein the image analysis device is configured to, every time after the at least one microscopic image block analysis result is obtained, determine whether the at least one microscopic image block analysis result meets a set condition, when the at least one microscopic image block analysis result meets the set condition, the output component is further configured to output the at least one microscopic image block analysis result, when the at least one microscopic image block analysis result does not meet the set condition the output component is further configured to output an overall analysis result after the overall analysis result is obtained;
wherein the set condition is whether abnormality exists or not or whether canceration exists or not.

5. An image analysis device, configured to analyze a pathological microscopic image in real time and comprising:

a microscopic image block acquisition component, establishing a data transmission connection with a microscopic image collection device and configured to acquire at least one microscopic image block corresponding to at least one position of a sample in real time at the same time when the microscopic image collection device sequentially shoots plurality of positions of the sample;
a microscopic image block analysis component, comprising a trained neural network model and configured to sequentially analyze the at least one microscopic image block corresponding to the at least one position to obtain at least one microscopic image block analysis result, each of the at least one microscopic image block analysis result comprising an analysis result, corresponding to each microscopic image block, of whether sample cells have a pathological abnormality or not;
an overall analysis component, configured to obtain an overall analysis result according to the at least one microscopic image block analysis result; and
an output component, configured to output the at least one microscopic image block analysis result and the overall analysis result, or the at least one microscopic image block analysis result, or the overall analysis result.

6. The image analysis device as claimed in claim 5, further comprising an image stitching component, configured to stitch a microscopic image block corresponding to each position of the sample to obtain an overall microscopic sample image, and the output component is further configured to output the overall microscopic sample image.

7. The image analysis device as claimed in claim 6, further comprising an image labeling component, configured to label a position of the pathological abnormality in the overall microscopic sample image according to the at least one microscopic image block analysis result, and the output component is further configured to output a labeled overall microscopic sample image.

8. The image analysis device as claimed in claim 5, further comprising a microscopic image block analysis result determination component, configured to, every time after the at least one microscopic image block analysis result is obtained, determine whether the at least one microscopic image block analysis result meets a set condition, when the at least one microscopic image block analysis result meets the set condition, the output component is further configured to output the at least one microscopic image block analysis result, when the at least one microscopic image block analysis result does not meet the set condition the output component is further configured to output an overall analysis result after the overall analysis result is obtained;
wherein the set condition is whether abnormality exists or not or whether canceration exists or not.

9. The image analysis device as claimed in claim 5, wherein the neural network model comprises a Convolutional Neural Network (CNN) model, and the trained neural network model is obtained by the following training steps:

obtaining training data, the training data being the microscopic image of the sample and labeling information corresponding to the microscopic image;
inputting the training data to the CNN model for training to obtain a trained CNN model, the CNN model comprising at least one convolutional layer, at least one pooling layer and at least one batch normalization layer and a loss function for training the CNN model being:

$$\mathrm{loss}(x, class) = -x[class] + \log\left(\sum_j \exp(x[j])\right)$$

or

$$\mathrm{loss}(x, class) = weight[class]\left(-x[class] + \log\left(\sum_j \exp(x[j])\right)\right),$$

wherein loss represents the loss function, weight represents a weight, x represents a predicted probability, class represents a corresponding class, and j represents serial numbers of all classes.

10. A real-time image analysis method, applied to real-time analysis of a pathological microscopic image and comprising:

acquiring at least one microscopic image block corresponding to at least one position of a sample in real time at the same time of sequentially shooting a plurality of positions of the sample;
sequentially inputting the at least one microscopic image blocks corresponding to the at least one position to a trained neural network model for analyzing to obtain at least one microscopic image block analysis result, each of the at least one microscopic image block analysis result comprising an analysis result, corresponding to each microscopic image block, of whether sample cells have a pathological abnormality or not;
obtaining an overall analysis result according to the at least one microscopic image block analysis result; and
outputting the overall analysis result.

11. The real-time image analysis method as claimed in claim 10, further comprising:

every time after the at least one microscopic image block analysis result is obtained, determining whether the at least one microscopic image block analysis result meets a set condition;
when the at least one microscopic image block analysis result meets the set condition, outputting the at least one microscopic image block analysis result;

when the at least one microscopic image block analysis result does not meet the set condition outputting an overall analysis result after the overall analysis result is obtained;
wherein the set condition is whether abnormality exists or not or whether canceration exists or not.

12. The real-time image analysis method as claimed in claim 10, wherein the neural network model comprises a Convolutional Neural Network (CNN) model, and the trained neural network model is obtained by the following training steps:

obtaining training data, the training data being the microscopic image of the sample and labeling information corresponding to the microscopic image;
inputting the training data to the CNN model for training to obtain a trained CNN model, the CNN model comprising at least one convolutional layer, at least one pooling layer and at least one batch normalization layer and a loss function for training the CNN model being:

$$\mathrm{loss}(x, class) = -x[class] + \log\left(\sum_j \exp(x[j])\right)$$

or

$$\mathrm{loss}(x, class) = weight[class]\left(-x[class] + \log\left(\sum_j \exp(x[j])\right)\right),$$

wherein loss represents the loss function, weight represents a weight, x represents a predicted probability, class represents a corresponding class, and j represents serial numbers of all classes.

13. The real-time image analysis method as claimed in claim 10, further comprising:
stitching a microscopic image block corresponding to each position of the sample to obtain an overall microscopic sample image.

14. The method as claimed in claim 13, further comprising:
labeling a position of the pathological abnormality in the overall microscopic sample image according to the at least one microscopic image block analysis result.

15. A non-transitory computer-readable storage medium, storing a computer-executable instruction, wherein when the computer-executable instruction is executed, a computer executes the method as claimed in claim 10.

**Fig. 1**

**Fig. 2**

**Fig. 3**

Training data is obtained — S401

A neural network model is trained by use of the training data to obtain the trained neural network model — S402

The trained neural network model is tested by use of test data — S403

**Fig. 4**

Fig. 5

S501
S502
S503
S504
S505
S506
S507

Fig. 6

Fig. 7

Europäisches
Patentamt

European
Patent Office

Office européen
des brevets

# EUROPEAN SEARCH REPORT

Application Number

EP 20 21 5060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/199392 A1 (GOOGLE LLC [US]) 17 October 2019 (2019-10-17) * page 13, lines 18-33; page 14, lines 6-11; page 29, lines 19-27; figure 11A * * page 7, lines 25-26; page 8, lines 17-23, 26-36; figure 1 * * page 9, l. 1-8 * * page 20, lines 13-14, 20-24, 28-31; figures 6, 11C * * page 33, lines 13-15, 19-20, 24-30 * * page 24, lines 13-20 *<br><br>----- | 1-15 | INV.<br>G06T7/00 |

TECHNICAL FIELDS
SEARCHED      (IPC)

G06T

The present search report has been drawn up for all claims

| Place of search | Date of completion of the search | Examiner |
|---|---|---|
| Munich | 28 April 2021 | Kollreider, Klaus |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...............................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 21 5060

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2021

| Patent document cited in search report | Publication date | Patent family member(s) | Publication date |
|---|---|---|---|
| WO 2019199392 A1 | 17-10-2019 | EP 3776458 A1<br>US 2021018742 A1<br>WO 2019199392 A1 | 17-02-2021<br>21-01-2021<br>17-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201911313396